# EUROPEAN PATENT SPECIFICATION

(11) **EP 1 998 833 B1**
(45) Date of publication and mention of the grant of the patent: **26.12.2012**
(21) Application number: 07759259.0
(22) Date of filing: 23.03.2007
(51) Int. Cl.: A61M 15/00, A61J 1/05, C23C 22/02, C23C 22/03, C23C 22/78, C23C 30/00

(54) **MEDICINAL FORMULATION CONTAINER WITH A TREATED METAL SURFACE**
BEHÄLTER FÜR MEDIZINISCHE FORMULIERUNG MIT BEHANDELTER METALLOBERFLÄCHE
CONTENANT POUR FORMULATION THÉRAPEUTIQUE DOTÉ D'UNE SURFACE MÉTALLIQUE TRAITÉE

(30) Priority: 24.03.2006 US 785823 P
(43) Date of publication of application: 10.12.2008
(73) Proprietor: 3M Innovative Properties Company, St. Paul, MN 55133-3427 (US)
(72) Inventor: BOARDMAN, Larry, D., Saint Paul, Minnesota 55133-3427 (US); JOHNSON, Peter, R., Saint Paul, Minnesota 55133-3427 (US); KORBA, Gary, A., Saint Paul, Minnesota 55133-3427 (US); MUELLER, Mark, E., Saint Paul, Minnesota 55133-3427 (US); PELLERITE, Mark, J., Saint Paul, Minnesota 55133-3427 (US); RAVICHANDRAN, Ravi, North Potomac, Maryland 20878 (US)
(74) Representative: Vossius & Partner
(86) International application number: PCT/US2007/064800
(87) International publication number: WO 2007/112312

(56) References cited:
- EP-A1- 1 241 113
- EP-A2- 0 520 649
- EP-A2- 1 693 053
- WO-A1-03/102003
- US-A- 5 217 743
- US-A- 5 738 920
- US-A1- 2005 042 553
- US-B1- 6 248 811
- US-B1- 6 248 811
- US-B2- 6 730 361

## Description

### Field

The present invention relates to medicinal formulation containers and methods of preparing the same. In particular, the present invention relates to metered dose inhalers.

### Background of the Invention

Metered dose inhalers are commonly used to treat a number of medical conditions. Typical medicinal formulations, i.e., solutions or suspensions of drug in a propellant, are housed within a pressurized canister and released in controlled amounts by a metering valve. It is important that the amount of drug released with each actuation of the metering valve be a consistent amount, so as to avoid over- or under-dosing of a patient.

In some instances, interaction of the medicinal formulation with the device components (e.g., canister, valve, etc.) may lead to variability in dosing. One example of such interactions may be of a chemical nature, such as through a drug degradation reaction that is catalyzed by the device material. Another example of such interactions may be of a physical nature, such as through adherence of suspended drug particles to the device. These interactions are generally dependent upon a number of factors, such as the type and shape of the material used in a device, the composition of the medicinal formulation, and the storage conditions of the device housing the medicinal formulation.

One well-known approach that attempts to eliminate or mitigate undesired material interactions is to provide a coating, such as a fluoropolymer, in the form of a film deposited on the base substrate of the device components.

### Summary

It has now been found that metal surfaces of a container, such as a metered dose inhaler, may be treated with very thin and highly rugged surface treatments that may reduce or eliminate undesirable interactions between the medicinal formulation and the device, while overcoming disadvantages with known methods. In particular, extremely thin surface treatments of approximately a single monolayer can be effective at reducing drug degradation, drug deposition, and/or corrosion caused by contact of a medicinal aerosol formulation with the interior surfaces of a metered dose inhaler.

In a first aspect, the present invention is a container suitable for use with a propellant-based medicinal formulation, the container comprising one or more treated metal surfaces that come into contact with the medicinal formulation, wherein the one or more treated metal surfaces are characterized by an organic surface treatment compound covalently bonded to the metal surface through a reactive head group selected from either phosphonic acid or carboxylic acid and wherein the organic surface treatment compound is further characterized by an exposed, substantially non-reactive tail group disposed away from the metal surface. In one embodiment, the container is a metered dose inhaler.

In an aspect, the present invention is a metered dose inhaler valve comprising a metering chamber, a stem, and a spring, one or more of which are metal and wherein at least one metal surface of the valve is characterized by an organic surface treatment compound covalently bonded to the metal surface through a reactive head group selected from either phosphonic acid or carboxylic acid and wherein the organic surface treatment compound is further characterized by an exposed, substantially non-reactive tail group disposed away from the metal surface.

In a fourth aspect, the present invention is a method of preparing a container having a treated metal surface suitable for use with a propellant-based medicinal formulation. A container having at least one metal surface and a treatment solution comprising an organic surface treatment compound comprising a reactive head group selected from either phosphonic acid or carboxylic acid and a substantially non-reactive tail group are provided. The treatment solution is applied to the metal surface of the container, allowed to remain in contact with the metal surface of the container for a period of time sufficient to allow the reactive head group to covalently bond with the metal surface, and then removed. Any non-covalently bound organic surface treatment compound may be optionally removed from the metal surface and the container is dried in order to provide a container having a metal surface characterized by an organic surface treatment compound covalently bonded to the metal surface through a reactive head group and wherein the organic surface treatment compound is further characterized by a substantially non-reactive tail group disposed away from the metal surface and wherein at least a portion of the non-reactive tail group is exposed to air.

The features and advantages of the present invention will be understood upon consideration of the detailed description of the preferred embodiment as well as the appended claims. These and other features and advantages of the invention may be described below in connection with various illustrative embodiments of the invention. The above summary of the present invention is not intended to describe each disclosed embodiment or every implementation of the present invention. The Figures and the detailed description which follow more particularly exemplify illustrative embodiments.

### Brief Description of the Drawings

Preferred embodiments of the invention will now be described in greater detail below with reference to the attached drawings, wherein:
FIG. I is a partial cross-sectional view of one embodiment of a device of the invention, wherein the valve stem is in the extended closed position.

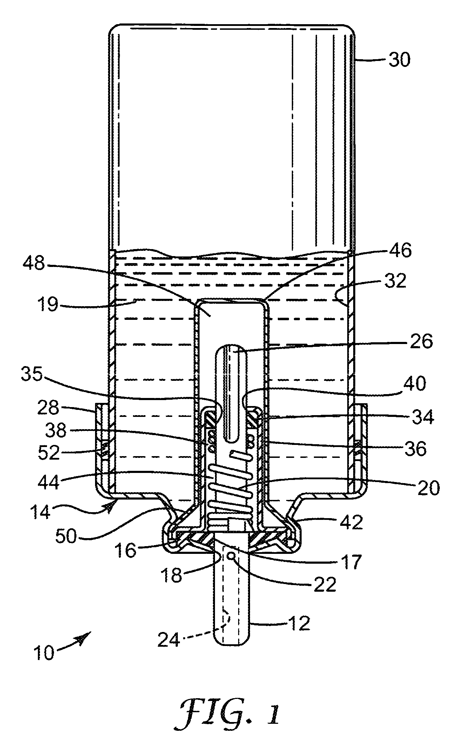

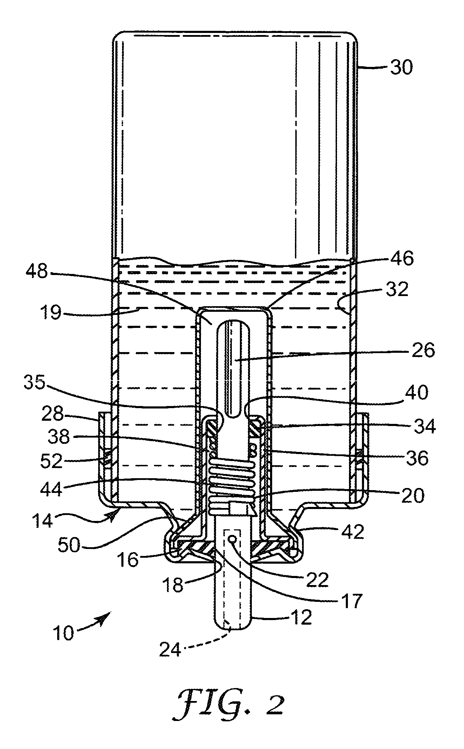

FIG. 2 is a partial cross-sectional view of the embodiment illustrated in FIG. 1, wherein the valve stem is in the compressed open position.

### Detailed Description

In the present invention, a metal surface of a container is characterized by an organic surface treatment compound covalently bonded to the metal surface through a reactive head group. The organic surface treatment compound is further characterized by a substantially non-reactive tail group that is disposed away from the metal surface.

Metal surfaces may be selected from any suitable metal, for example, aluminum, steel, stainless steel, metal oxides, and alloys and mixtures thereof. In one embodiment, it may be desirable to apply the organic surface treatment compound to the native oxide surface layer of a metallic substrate.

Suitable organic surface treatment compounds have a reactive head group that may form a covalent bond with a metal surface and a substantially non-reactive tail group. The term "head" group is generally used to encompass any reactive group that may bond with a metal surface and thereby leave a remaining portion of the molecule, i.e., the "tail" group, disposed away from the metal surface. In one embodiment, the compounds may be generally linear with the head group at one end and the tail group at the opposing end of the molecule. Alternatively, the head group may be at a generally central location in the molecule with two or more tail groups extending away from the head group. In still another alternative, a compound may have a head group at two or more terminal points with the central portion of the compound serving as the tail group. For example, a linear compound may have a head group at each end, both of which can form a covalent bond with a metal surface. The central, tail group may then extend away from the metal surface in (at least schematically) a "U" shape.

The reactive head groups include a chemical functionality that is easily capable of reacting with a metal surface, such as an acidic functionality. Typical acid functional groups that may be used include carboxylic acid, phosphonic acid, and sulfonic acid. In one embodiment, the reactive head group has sufficient reactivity such that it will react with a metal surface under conditions of ambient temperature and pressure. Phosphonic acid and carboxylic acid are preferred functionalities for the reactive head group. Although not wishing to be bound by theory, it is believed that such acid head group functionalities are advantageous when compared to ester head group functionalities, such as a phosphoric ester, since the ester linkage may be easily broken during or after surface modification, thus potentially leading to incomplete surface coverage and/or increased amounts of impurities that are not covalently bonded to the metal surface. Likewise, the acid head group functionalities of the present invention are also believed to be advantageous when compared to silane head group functionalities, since the silane head group functionality may be susceptible to reacting with adjacent silane head groups instead of covalently bonding with the metal surface. The tail group has a generally non-reactive character. By substantially non-reactive, it should be understood that this is not meant to imply that the tail group is completely inert, but rather that the tail group is generally non-reactive under the ordinary conditions to which a metered dose inhaler may be exposed. These conditions may vary somewhat from inhaler to inhaler and may depend, for example, on the composition of the medicinal formulation and the expected shelf life of the inhaler. A substantially non-reactive tail group will undergo no more than an insignificant amount of reaction with the medicinal formulation. In particular, no more than 10% of the drug present in a medicinal formulation will react with the tail group during storage at room temperature for a period of 6 or more months.

Typical non-reactive tail groups include linear, branched, or cyclic alkanes, ethers, fluoroalkanes, fluoroethers, and combinations thereof. The tail group may vary in size, but will typically range from a very low molecular weight entity, such as a perfluoropropyl group, to somewhat larger molecular weight entities. The tail groups typically have a carbon or carbon and oxygen backbone with 3 or more atoms in the backbone, often with 6 or more atoms in the backbone, and sometimes with 12 or more atoms in the backbone. The tail groups typically have a carbon or carbon and oxygen backbone with 50 or less atoms in the backbone, often with 40 or less atoms in the backbone, and sometimes with 22 or less atoms in the backbone. In one embodiment, the tail group comprises 3 to 22 carbons, and may comprise a linear alkyl having 3 to 22 carbons or a branched alkyl having 3 to 22 carbons. In one embodiment, the tail group is a linear alkyl having 3 to 22 carbons or a branched alkyl having 3 to 22 carbons. In one embodiment, the tail group comprises fluorine, and in some instances may terminate with a linear or branched perfluoroalkyl having 1 to 4 carbons, 1 to 6 carbons, or 4 carbons. In one embodiment, the tail group may have a "blocked" structure comprising a first chemical structure, such as an alkane, with one end directly connected to the head group and a second chemical structure, such as a perfluoroalkane connected to the other end of the first chemical structure (e.g., 1-phosphono-11-(nonafluorobutyl)undecane). Although not wishing to be bound by theory, it is believed that a relatively short tail group (e.g., less than about 22 carbons) aids in the formation of a complete and robust surface treatment.

In one embodiment, the organic surface treatment compounds may have the formula X-R₁-R₂, wherein X is 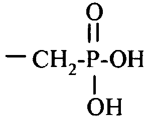 or -COOH;
R₁ is linear or branched alkyl having 0 to 22 carbons and optionally substituted by -O-;
R₂ is linear or branched perfluoroalkyl having 0 to 6 carbons; and
wherein R₁ and R₂ together comprise at least 3 and no more than 22 carbons. In one embodiment X is 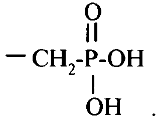 In one embodiment, R₁ has 0 carbons, i.e., R₁ is absent. In another embodiment, R₂ has 0 carbons, i.e., R₂ is absent. In one embodiment, R₁ is linear or branched alkyl having 3 to 22 carbons. In one embodiment, R₂ is linear or branched perfluoroalkyl having 2 to 6 carbons. In another embodiment, R₁ is substituted by -O-, i.e., R₁ contains one or more ether linkages.

The amount of organic surface treatment compounds applied to a metal surface is preferably sufficient to form a monolayer coating. That is, a single layer of the organic surface treatment compound will be present on the metal surface. Depending on the type of coating conditions used, the organic surface treatment compound may be applied in an amount that only provides a partial monolayer or in an amount in excess of one monolayer. Where the organic surface treatment compound is provided in an amount in excess of a monolayer, it is believed that the excess material is at most only weakly bound and may be removed easily by rinsing with an appropriate solvent. Thus the organic surface treatment compound will be bound to the metal surface through the reactive head group and the unreactive tail group will be exposed. That is, for a treated metal part in isolation, such as the treated internal surface of a medicinal aerosol canister that is not yet filled with medicinal formulation, the tail group will be exposed to air, whereas the tail group will be exposed to medicinal formulation once the medicinal aerosol canister is filled with medicinal formulation. The portion of the tail group exposed will depend upon the type of molecule employed, as well as the packing or alignment of the molecule on the metal surface. In one embodiment, the head groups of the surface treatment compounds may be closely packed enough so that the tail groups extend from the metal surface in a brush-like fashion. In another embodiment, the head groups may be less tightly packed so that the tail groups can take on a partly coiled structure extending from the surface, thereby exposing a greater portion of the tail group to air or medicinal formulation. Desirably, the organic surface treatment compound forms a monolayer (e.g., a self-assembled monolayer) on the surface of the metal substrate. In one embodiment, the organic surface treatment compound may form a substantially complete monolayer on the surface of the metal substrate. For purposes of definition, a substantially complete monolayer surface treatment will cover greater than about 95% of any reactive sites on the metal surface. The layer of organic surface treatment compound may be of any thickness, but after rinsing away any excess unbound material and drying, the thickness is typically in the range of from about 0.5 to about 10 nanometers (nm), often in the range of from about 1 to about 5 nm, and sometimes in the range of from about 1 to about 2.5 nm. In one embodiment, the treated metal surface is free of or has substantially no non-covalently bonded organic surface treatment compound. For purposes of definition, substantially all of the organic surface treatment compound is considered to be covalently bonded to the metal surface if greater than about 95% by weight of the total amount of organic surface treatment compound present on the metal surface is covalently bonded to the surface. The total amount of organic surface treatment material is generally quite small and is preferably tightly bound to the metal surface, which preferably minimizes or eliminates the ability of a medicinal formulation to extract impurities from the treated metal surface. A typical metered dose inhaler canister has about 30 cm² of surface area that may come into contact with medicinal formulation. In one embodiment, the total amount of organic surface treatment compound on the surface of the canister is less than about 50 µg, less than about 30 µg, or less than about 10 µg. In another embodiment, the total amount of organic surface treatment compound on the surface of the canister is less than about 5 µg, less than about 2 µg, and sometimes less than about 1 µg.

In one embodiment, a treatment solution comprising the organic surface treatment compound is applied to at least one metal surface of a container and allowed to remain in contact with the metal surface for a period of time sufficient to allow the reactive head group to covalently bond with the metal surface. Exemplary methods for applying the organic surface treatment compounds to a substrate include, for example, spraying, dip coating, wiping, and spin coating of a dilute (e.g., a 0.1 weight percent) solution of the compound in an organic solvent such as ethanol, methanol or isopropyl alcohol. Any non-covalently bound organic surface treatment compound may be optionally rinsed from the metal surface. The container is then removed from contact with the treatment solution and dried to provide a container with a treated metal surface. It may be desirable to pre-treat the metal surface to remove any weak boundary layer of material that may be present. For example, certain metal surfaces may be partially or fully covered by residual process oils that are used to facilitate the machining process. Such oils are desirably removed by rinsing or soaking a metal part in a solvent capable of removing the process oil, thereby exposing the metal surface. In one embodiment, ultrasonic agitation may be applied during the rinsing or soaking pre-treatment to facilitate removal of any weak boundary layer material.

The individual organic surface treatment compounds covalently bound to a metal surface generally interact with each other only through van der Waals forces. That is, adjacent organic surface treatment compounds are not crosslinked or otherwise covalently bound to each other.

In one embodiment the container is a metered dose inhaler device as shown in FIGS. 1 and 2. FIG. 1 shows device 10 comprising valve stem 12, casing member 14, and diaphragm 16. The casing member has walls defining casing aperture 18, and the diaphragm has walls defining diaphragm aperture 17. The valve stem passes through and is in slidable sealing engagement with the diaphragm aperture. The diaphragm is also in sealing engagement with casing member 14.

The illustrated embodiment is a device for use with pharmaceutical formulations. The diaphragm in the illustrated embodiment is a single piece of a thickness sufficient to form an effective seal with the casing member, preferably about 0.125 mm (0.005 inch) to about 1.25 mm (0.050 inch). It has an outside diameter of about 8.6 mm (0.340 inch), and an inside diameter sufficient to form an effective seal with the valve stem. As valve stems having an outside diameter of about 2.79 mm (0.110 inch) are commonly used, suitable diaphragm inside diameter can be in the range of about 2.03 mm (0.080 inch) to about 2.67 mm (0.105 inch). Diaphragm dimensions suitable for use with other general types of devices can be easily selected by those skilled in the art.

Valve stem 12 is in slidable engagement with diaphragm aperture 17. Helical spring 20 holds the valve stem in an extended closed position as illustrated in FIG. 1. Valve stem 12 has walls defining orifice 22 which communicates with exit chamber 24 in the valve stem. The valve stem also has walls defining channel 26.

In the illustrated embodiment casing member 14 comprises mounting cup 28 and canister body 30 and defines formulation chamber 32. The illustrated embodiment further comprises tank seal 34 having walls defining tank seal aperture 35, and metering tank 36 having inlet end 38, inlet aperture 40, and outlet end 42. The metering tank also has walls defining metering chamber 44 of predetermined volume (e.g., 50 µL). Outlet end 42 of metering tank 36 is in sealing engagement with diaphragm 16, and valve stem 12 passes through inlet aperture 40 and is in slidable engagement with an elastomeric tank seal 34.

When device 10 is intended for use with a suspension aerosol formulation it may further comprise a retaining cup 46 fixed to mounting cup 28 and having walls defining retention chamber 48 and aperture 50. When intended for use with a solution aerosol formulation retaining cup 46 is optional. Also illustrated in device 10 is sealing member 52 in the form of an O-ring that substantially seals formulation chamber 32 defined by mounting cup 28 and canister body 30.

Operation of device 10 is illustrated in FIGS. I and 2. In FIG. 1, the device is in the extended closed position. Aperture 50 allows open communication between retention chamber 48 and formulation chamber 32, thus allowing the aerosol formulation to enter the retention chamber. Channel 26 allows open communication between the retention chamber and metering chamber 44 thus allowing a predetermined amount of aerosol formulation to enter the metering chamber through inlet aperture 40. Diaphragm 16 seals outlet end 42 of the metering tank.

FIG. 2 shows device 10 in the compressed open position. As valve stem 12 is depressed channel 26 is moved relative to tank seal 34 such that inlet aperture 40 and tank seal aperture 35 are substantially sealed, thus isolating a metered dose of formulation within metering chamber 44. Further depression of the valve stem causes orifice 22 to pass through aperture 18 and into the metering chamber, whereupon the metered dose is exposed to ambient pressure. Rapid vaporization of the propellant causes the metered dose to be forced through the orifice, and into and through exit chamber 24. Device 10 is commonly used in combination with an actuator that facilitates inhalation of the resulting aerosol by a patient.

One or more of the metal surfaces that come into contact with the medicinal formulation are treated metal surfaces as described above. This includes for example, the inner surface of the canister body 30 or casing member 14, valve components, such as the valve stem 12, helical spring 20, metering tank 36, or retaining cup 46.

One embodiment of the device of the present invention is a metered dose configuration substantially as described above and illustrated in FIGS. 1 and 2. Other particular configurations, metered dose or otherwise, well known to those skilled in the art are suitable. For example the devices described in U.S. Patent Nos. 4,819,834 (Thiel), 4,407,481 (Bolton), 3,052,382 (Gawthrop), 3,049,269 (Gawthrop), 2,980,301 (DeGorter), 2,968,427 (Meshberg), 2,892,576 (Ward), 2,886,217 (Thiel), and 2,721,010 (Meshberg) involve a valve stem, a diaphragm, and a casing member in the general relationship described herein.

Treated metal surfaces of the present invention are also suitable for use in other metered dose devices comprising a medicinal composition, such as those disclosed in U. S. Patent Nos. 5,772,085 (Bryant et al.), 6,454,140 (Jinks), 6,644,517 (Thiel et al.), 6,640,805 (Castro et al.), U.S. Published Patent Applications Nos. 2003/010794 (Herdtle et al.), 2003/127464 (Bryant et al.), 2003/121935 (Arsenault et al.), 2004/139965 (Greenleaf et al.), and 2004/139966 (Hodson). In one embodiment, the container may be a larger vessel that is part of a filling system suitable for manufacturing bulk supplies of medicinal formulation. Surface treatment of part or all of the vessel surfaces may aid in reducing drug deposition during the manufacturing process, thereby eliminating or reducing the need to initially use an overage of drug in order to prepare individual devices having a desired dosage. It may also be desirable to treat other metal surfaces in the filling system, such as any tubing or parts used for stirring the formulation.

Examples of suitable propellants for use in aerosol formulations of the present invention include 1,1,1,2-tetrafluoroethane (HFA-134a, also known as HFC-134a), 1,1,1,2,3,3,3-heptafluoropropane (HFA-227, also known as HFC-227), fluorotrichloromethane, dichlorodifluoromethane, and 1,2-dichlorotetrafluoroethane, and mixtures thereof. Preferred propellants are 1,1,1,2-tetrafluoroethane (HFA-134a), 1,1,1,2,3,3,3-heptafluoropropane (HFA-227), and mixtures thereof. In one embodiment, the propellant is 1,1,1,2,3,3,3-heptafluoropropane (HFA-227).

Preferred medicinal formulations generally comprise HFA- 134a, HFA-227, or a mixture thereof in an amount effective to function as an aerosol propellant, a drug having local or systemic action and suitable for use by inhalation, and any optional formulation excipients. Optional excipients include cosolvents (e.g., ethanol, water) and surfactants (e.g., oleic acid, sorbitan esters, polyoxyethylenes, glycols) and others known to those skilled in the art. In one embodiment, medicinal formulations of the present invention comprise from less than 25% ethanol by weight of the total formulation, often less than 15%, and sometimes less than 10%. In one embodiment, medicinal formulations of the present invention comprise more than 1% ethanol by weight of the total formulation. In one embodiment, medicinal formulations of the present invention are free or substantially free of cosolvents. In one embodiment, medicinal formulations of the present invention are free or substantially free of surfactants. In another embodiment, medicinal formulations of the present invention consist essentially of 1,1,1,2,3,3,3-heptafluoropropane and drug.

As used herein, the term "drug," includes its equivalents, "bioactive agent," and "medicament" and is intended to have its broadest meaning as including substances intended for use in the diagnosis, cure, mitigation, treatment or prevention of disease, or to affect the structure or function of the body. The drugs can be neutral or ionic. Preferably, they are suitable for oral and/or nasal inhalation. Delivery to the respiratory tract and/or lung, in order to effect bronchodilation and to treat conditions such as asthma and chronic obstructive pulmonary disease, is preferably by oral inhalation. Alternatively, to treat conditions such as rhinitis or allergic rhinitis, delivery is preferably by nasal inhalation. Preferred drugs are asthma, allergy, or chronic obstructive pulmonary disease medications.

Suitable drugs include, for example, antiallergics, anticancer agents, antifungals, antineoplastic agents, analgesics, bronchodilators, antihistamines, antiviral agents, antitussives, anginal preparations, antibiotics, anti-inflammatories, immunomodulators, 5-lipoxygenase inhibitors, leukotriene antagonists, phospholipase A2 inhibitors, phosphodiesterase IV inhibitors, peptides, proteins, steroids, and vaccine preparations. Exemplary drugs include adrenaline, albuterol, atropine, beclomethasone dipropionate, budesonide, butixocort propionate, ciclesonide, clemastine, cromolyn, epinephrine, ephedrine, fenoterol, fentanyl, flunisolide, fluticasone, formoterol, ipratropium bromide, - isoproterenol, levalbuterol, lidocaine, mometasone, morphine, nedocromil, pentamidine isoethionate, pirbuterol, prednisolone, resiquimod, salmeterol, terbutaline, tetracycline, triamcinolone, and pharmaceutically acceptable salts and solvates thereof, and mixtures thereof. In one embodiment, the drug is a β2-adrenoreceptor agonist, such as albuterol, fenoterol, formoterol, isoproterenol, levalbuterol, pirbuterol, salmeterol, and pharmaceutically acceptable salts and solvates thereof, and mixtures thereof. A group of preferred drugs include albuterol, beclomethasone dipropionate, flunisolide, fluticasone, formoterol, ipratropium bromide, pirbuterol, salmeterol, and pharmaceutically acceptable salts and solvates thereof, and mixtures thereof.

The drug is generally present in the medicinal formulation in an amount sufficient to provide a predetermined number of therapeutically effective doses by inhalation, which can be easily determined by those skilled in the art considering the particular drug in the formulation.

In one embodiment, the treated surface may prevent undesirable chemical reactions between the base metal and one or more components of a medicinal formulation. For example, a number of steroid drugs, such as those described in U. S. Patent No. 6,315,985 (Wu et al.), are relatively susceptible to chemical degradation when exposed to untreated aluminum oxide surfaces. Medicinal formulations in metered dose inhalers having the surface treatments described above applied to the internal (or formulation contacting) surfaces will preferably undergo less drug degradation on storage than the medicinal formulation would in a like inhaler lacking the surface treatment.

In one embodiment, the treated surface may prevent the occurrence of corrosion in a metered dose inhaler. For example, metered dose inhalers containing dissimilar metals, such as an aluminum canister and a valve comprising one or more steel components may be susceptible to corrosion, as the medicinal formulation contacting both metals may act as an electrochemical cell. Such potential corrosion may be inhibited by providing a surface treatment for one of the dissimilar metals, so that the medicinal formulation is no longer in contact with two untreated metal surfaces. In one embodiment, the valve components may be surface treated and the canister untreated. In one embodiment, the canister may be surface treated and the valve components untreated. In one embodiment, both the valve components and the canister may be surface treated. Metered dose inhalers having the surface treatments described above applied to the internal (or formulation contacting) surfaces will preferably undergo less corrosion when exposed to a particular medicinal formulation than a like inhaler lacking the surface treatment.

In one embodiment, the treated surface may prevent drug from adhering to container surfaces, such as the interior surface of a metered dose inhaler canister or the surfaces of a metered dose inhaler valve that come into contact with medicinal formulation. Such adherence of drug may be seen, for example, where particulate drug suspended in a medicinal formulation can become adsorbed or otherwise adhere to an untreated metal surface. In one embodiment, all of the metal valve components may be surface treated. In another embodiment, the metering tank may be surface treated and the remaining metal valve components untreated. Medicinal formulations in metered dose inhalers having the surface treatments described above applied to the internal (or formulation contacting) surfaces will preferably undergo less adhesion of drug to the internal surfaces than the medicinal formulation would in a like inhaler lacking the surface treatment.

### Examples

All parts, percentages and ratios in the following examples are by weight unless stated otherwise. "Room temperature" in the following examples means approximately 20 °C to 24 °C.

### Surface Chemical Composition Measurement

The surface chemical composition of treated aluminum oxide surfaces was determined by analyzing each aluminum oxide sample using X-ray Photoelectron Spectroscopy (XPS or ESCA). All spectra were taken with a Kratos Axis Ultra™ XPS system utilizing a monochromatic AlK_{α} x-ray excitation source and a hemispherical electron energy analyzer operated in a constant pass energy mode. The photoelectron collection (take-off) angle was 90° measured with respect to the analyzer lens axis with a ± 10° solid angle of acceptance. Compositions were calculated from survey spectra using linear backgrounds.

### Valve Deposition Measurement

Metered dose inhalers (MDIs) were fired 122 times to waste. The MDI was shaken for not less than 3 seconds prior to each actuation with a delay of at least 30 seconds between successive actuations. An additional 5 shots were then fired to waste with the MDI in a valve up orientation to clean out the valve. The metering valves were then removed from the MDIs and the outside of the stem cleaned to remove any residual drug adhering to the outer surface. The valve was immersed in diluent (acetonitrile: water 75:25 v/v) and sonicated for approximately 2 hours followed by shaking for at least 30 minutes. An aliquot was removed and analyzed for drug content by conventional HPLC analysis (albuterol sulfate and pirbuterol acetate: 55% 100 mM triethylamine phosphate with 5 mM sodium dodecyl sulfate, pH 2.5 / 45% methanol v:v, UV detection at 225 nm; flunisolide hemihydrate: 60% water / 40% acetonitrile v:v, UV detection at 244 nm). Results are reported as an average of 5 to 8 replicates unless otherwise noted.

### Example 1

The internal surface of an aluminum metered dose inhaler canister was treated with compound A1 (n-C₄F₉-(CH₂)₁₁-PO₃H₂) which may be prepared as described in Example 2 ofU. S. Patent No. 6,824,882, as follows. The canister was initially soaked in heptane (analytical reagent grade) for 30 minutes while exposed to ultrasonic agitation. The canister was then rinsed with heptane (analytical reagent grade) for one minute followed by a rinse with isopropanol (analytical reagent grade) and allowed to air dry. The canister was then immersed in a 0.1 % w/w solution of A1 in methyl tertiary butyl ether (analytical reagent grade) for 30 minutes while exposed to ultrasonic agitation. The canister was then rinsed in methyl tertiary butyl ether (analytical reagent grade) for one minute and allowed to air dry.

Fluticasone propionate was added to HFA-134A propellant to prepare a medicinal formulation with a fluticasone propionate concentration of 0.0083% w/w. Approximately 13 g of medicinal formulation was added to the treated canister which was sealed with an untreated aluminum blind ferrule (i.e., a casing member lacking valve components). The canister was stored in an upright position after filling to prevent contact of the medicinal formulation with the untreated aluminum surface of the blind ferrule. The solution was stored in the canister for 3 days at room temperature. The amount of fluticasone propionate adsorbed to the canister was 2.4 % (+/- 1.1%) measured according to the method described above.

### Example 2

A canister was prepared and tested as described in Example 1 with the exception that the canister was filled with a 0.0083% w/w formulation of fluticasone propionate in HFA-227. The solution was stored in the canister for 3 days at room temperature. The amount of fluticasone propionate adsorbed to the canister was 9.7 % (+/- 6.5%) measured according to the method described above.

### Comparative Example 1

An untreated aluminum canister was filled with the medicinal formulation of Example 1. The solution was stored in the canister for 3 days at room temperature. The amount of fluticasone propionate adsorbed to the canister was 23.2 % (+/- 1.5%) measured according to the method described above.

### Comparative Example 2

An untreated aluminum canister was filled with the medicinal formulation of Example 2. The solution was stored in the canister for 3 days at room temperature. The amount of fluticasone propionate adsorbed to the canister was 35.5 % (+/- 1.8%) measured according to the method described above.

### Example 3

Aluminum oxide powder (Aldrich, neutral, activated Brockmann I, 150 mesh), which mimics aluminum oxide existing on the inner surface of aluminum containers was surface treated by being immersed in a 0.1 % w/w solution of A 1 in methyl tertiary butyl ether (analytical reagent grade) for 30 minutes while exposed to ultrasonic agitation. The methyl tertiary butyl ether solution was decanted and the powder was allowed to air dry. A triamcinolone stock solution (4mM) was prepared by adding triamcinolone acetonide to ethanol. Approximately 0.1 g of treated powder was then placed in a vial containing 2 mL of triamcinolone stock solution and stored at 70°C. An extract of the triamcinolone solution was taken after one day of storage and analyzed using high performance liquid chromatography to determine remaining drug content. The amount of drug remaining is reported (in Table 1) as a percentage of the initial amount of drug present and was determined as the average of 3 independent measurements.

Surface chemical composition of the aluminum oxide powder after exposure to the drug solution was measured using X-ray photoelectron spectroscopy. The percentage of the aluminum oxide powder remaining chemically treated after exposure to drug solution is shown in Table 1.

### Examples 4-10

Aluminum oxide powder was surface treated and tested as described above with a variety of other organic surface treatment compounds. The type of compound, the amount of triamcinolone acetonide (TA) remaining after 1 day storage, and the percentage of aluminum oxide (AO) powder remaining chemically treated after exposure to drug solution in shown in Table 1.

The following abbreviations are used in Table 1:
FC-23 = FC-23 Fluorad™ Fluorochemical Acid, perfluorobutyric acid, C₃F₇COOH (available from 3M), surface treatment using methyl tertiary butyl ether solution.
A2 = 1-phosphono-3,7,11,15-tetramethylhexadecane, CH₃-(CH(CH₃)CH₂CH₂CH₂)₃CH(CH₃)CH₂CH₂-PO₃H₂ which may be prepared as described in U. S. Patent No. 6,433,359, surface treatment using ethanol solution.
A3 = 1-phosphonohexadecane, n-C₁₆H₃₃-PO₃H₂ (available from Oryza Laboratories, Chelmsford, MA), surface treatment using methanol solution.
A4 = n-C₄F₉-(CH₂)₁₀-COOH, which may be prepared as described in U. S. Patent Application Publication No. 2004/0241396 (Jing et al.), surface treatment using ethyl acetate solution
A5 = Phytanyl carboxylic acid, CH₃-(CH(CH₃)CH₂CH₂CH₂)₃CH(CH₃)CH₂-COOH, which may be prepared as described in "Convenient Highly Stereoselective Syntheses of (3R,7R,11R)- and (3S,7R,11R)- 3,7,11,15-Tetramethylhexadecanoic Acid (Phytanic Acid) and the Corresponding 3,7,11,15-Tetramethylhexadecan-1-ols, L.R. Sita, Journal of Organic Chemistry 1993, 58, 5285-5287, surface treatment using ethanol solution.
KRY = Krytox™ 157 FSL, polyfluoroether monocarboxylic acid, C₃F₇-O-[CF(CF₃)CF₂-O]₁₃-CF(CF₃)-CO₂H (available from DuPont), surface treatment using methanol solution.
FOM = Fomblin™ Z-Diacid, polyfluoroether diol, HO₂C-CF₂-O-(CF₂O)₁₁- (CF₂CF₂-O)₁₁-CF₂-CO₂H (available from Ausimont), surface treatment using methanol solution.

| Table 1 | | | |
|---|---|---|---|
| Ex. No. | Cmpd. Type | % TA remaining | % AO treated - final |
| 3 | Al | 86.2 (+/- 3.7) | 100 |
| 4 | FC-23 | 91.7 (+/- 1.5) | 100 |
| 5 | A2 | 97.4 (+/- 0.1) | 100 |
| 6 | A3 | 93.2 (+/- 3.6) | 100 |
| 7 | A4 | 87.8 (+/- 0.6) | 100 |
| 8 | A5 | 71.5 (+/- 3.2) | 100 |
| 9 | KRY | 96.3 (+/- 0.7) | 95 |
| 10 | FOM | 96.7 (+/- 0.1) | 96 |
| C3 | None | 11.5 (+/- 1.2) | NA |

### Comparative Example 3 (C3)

Approximately 0.1 g of untreated aluminum oxide powder was placed in a vial containing 2 mL of triamcinolone stock solution and stored at 70°C. An extract of the triamcinolone solution was taken after one day of storage and analyzed using high performance liquid chromatography to determine remaining drug content. The amount of drug remaining is shown in Table 1.

### Example 11

The internal surface of a stainless steel metered dose inhaler canister was treated with compound A1 (n-C₄F₉-(CH₂)₁₁-PO₃H₂) as follows. The canister was initially soaked in heptane (analytical reagent grade) for 30 minutes while exposed to ultrasonic agitation. The canister was then rinsed with heptane (analytical reagent grade) for one minute followed by a rinse with isopropanol (analytical reagent grade) and allowed to air dry. The canister was then immersed in a 0.1 % w/w solution of A1 in methyl tertiary butyl ether (analytical reagent grade) for 30 minutes while exposed to ultrasonic agitation. The canister was then rinsed in methyl tertiary butyl ether (analytical reagent grade) for one minute and allowed to air dry.

A triamcinolone stock solution (2 mM) was prepared by adding triamcinolone acetonide to ethanol. The canister was filled with 5 mL of the triamcinolone stock solution, sealed and stored at 70°C. An extract of the triamcinolone solution was taken after one day of storage and analyzed using high performance liquid chromatography to determine remaining drug content. The amount of triamcinolone acetonide remaining after 1 day was 94.9 %. An extract of the triamcinolone solution was taken after 7 days of storage and analyzed using high performance liquid chromatography to determine remaining drug content. The amount of triamcinolone acetonide remaining after 7 days was 83.0 %.

### Comparative Example 4

An untreated stainless steel canister was filled with the medicinal formulation of Example 11. The amount of triamcinolone acetonide remaining after 1 day was 88.0 %. An extract of the triamcinolone solution was taken after 7 days of storage and analyzed using high performance liquid chromatography to determine remaining drug content. The amount of triamcinolone acetonide remaining after 7 days was 18.0 %.

### Example 12

The internal surface of a stainless steel filling vessel used for filling metered dose inhaler canisters was surface treated with compound A1 (n-C₄F₉-(CH₂)₁₁-PO₃H₂). The vessel was initially soaked in heptane (analytical reagent grade) for 30 minutes while exposed to ultrasonic agitation. The vessel was then rinsed with heptane (analytical reagent grade) for one minute followed by a rinse with isopropanol (analytical reagent grade) and allowed to air dry. The vessel was then immersed in a 0.1 % w/w solution of A1 in methyl tertiary butyl ether (analytical reagent grade) for 30 minutes while exposed to ultrasonic agitation. The vessel was then rinsed in methyl tertiary butyl ether (analytical reagent grade) for one minute and allowed to air dry. Additional components of the filling system that contact medicinal formulation during the filling process (solenoid, tubing, stirring paddles, mixing baffles) were treated in the same manner as the vessel.

HFA-227a (600.7 g) was chilled and added to the filling vessel. Fluticasone propionate (0.0499 g) was added to the filling vessel and stirred using a high shear mixer at 3000 rpm for 15 minutes to provide a fluticasone propionate suspension in HFA-227a with a nominal concentration of 0.0083% w/w. Forty metered dose inhaler canisters were filled with approximately 13 g of formulation. A valve was crimped onto each canister immediately after filling. The total drug content (i.e., drug suspended in formulation, as well as drug adhered to the canister) of 6 representative canisters (numbers 9, 11, 13, 14, 16, and 18 in the filling sequence) was analyzed using high performance liquid chromatography. A target total drug content was calculated based on the exact amount of formulation added to each canister and the nominal concentration in the filling vessel. The target total drug content in each canister was approximately 0.00108 g. The resulting average total drug content in the filled canisters was 2% less than the target total drug content.

### Comparative Example 5

Metered dose inhalers were prepared as in Example 12 with the exception that an untreated stainless steel filling system was used. The resulting average total drug content in the filled canisters was 58% less than the target total drug content.

### Example 13

The internal surfaces of aluminum, 50 µL metered dose inhaler metering valves were treated with compound A1 (n-C₄F₉-(CH₂)₁₁-PO₃H₂) as follows.

Individual valve components (stem, tank, spring, ferrule, and bottle emptier) were initially soaked in isopropanol (analytical reagent grade) for 30 minutes while exposed to ultrasonic agitation. Excess solvent was removed and the components were air dried.

The valve components were then immersed in a 0.1 % w/w solution of A 1 in methyl tertiary butyl ether (analytical reagent grade) in a sealed flask with a vacuum valve that was placed in a sonicator bath. The flask was sonicated and a vacuum was pulled until air escaped from the small bore in the stems (at least 2 minutes). The vacuum valve was then closed and the sample maintained under vacuum and sonication for an additional 30 minutes. Excess coating solution was then removed and the metering valves were rinsed and sonicated under vacuum in fresh isopropanol (analytical reagent grade) for 15 minutes. Excess rinsing solution was then removed and the metering valves were rinsed and sonicated under vacuum a second time in fresh isopropanol (analytical reagent grade) for 15 minutes. Excess rinsing solution was again removed and the metering valves were dried at 50 °C for at least 12 hours. The components were then assembled to form treated metered dose inhaler metering valves.

Albuterol sulfate was added to HFA-134A propellant to prepare a medicinal formulation with an albuterol sulfate concentration of 0.05% w/w. Approximately 8.7 g of medicinal formulation was added to an aluminum canister with an internal coating of FEP (tetrafluoroethylene/hexafluoropropylene copolymer). The canister was then sealed by crimping a treated valve onto the canister to form an MDI. The MDIs were stored in a valve down orientation after filling to ensure contact of the formulation with the valve. The amount of albuterol sulfate adsorbed to the valve was 112 (+/-25) µg measured according to the valve deposition method described above. Visual observation of the inside surface of the metering tanks showed considerably less drug deposition than the corresponding metering tanks from Comparative Example 6 using untreated valves.

### Comparative Example 6

Metered dose inhalers were prepared as in Example 13, with the exception that the metering valves used were untreated. The amount of albuterol sulfate adsorbed to the valve was 138 (+/-24) µg measured according to the method described above.

### Example 14

MDIs were prepared as in Example 13, with the exception that the medicinal formulation used was 0.05% w/w albuterol sulfate in HFA-227 propellant and approximately 10.2 g of medicinal formulation was added to each canister. The amount of albuterol sulfate adsorbed to the valve was 134 (+/-54) µg measured according to the method described above. Visual observation of the inside surface of the metering tanks showed considerably less drug deposition than the corresponding metering tanks from Comparative Example 7 using untreated valves.

### Comparative Example 7

Metered dose inhalers were prepared as in Example 14, with the exception that the metering valves used were untreated. The amount of albuterol sulfate adsorbed to the valve was 167 (+/-28) µg measured according to the method described above.

### Example 15

MDIs were prepared as in Example 14, with the exception that albuterol sulfate was added to a mixture of ethanol and HFA-227 propellant to prepare a medicinal formulation with 0.05% w/w albuterol sulfate and 10% w/w ethanol in HFA-227 propellant. The amount of albuterol sulfate adsorbed to the valve was 67 (+/-18) µg measured according to the method described above. Visual observation of the inside surface of the metering tanks showed minimal drug deposition in the metering tanks.

### Comparative Example 8

Metered dose inhalers were prepared as in Example 15, with the exception that the metering valves used were untreated. The amount of albuterol sulfate adsorbed to the valve was 75 (+/-9) µg measured according to the method described above. Visual observation of the inside surface of the metering tanks showed minimal drug deposition in the metering tanks.

### Example 16

MDIs were prepared as in Example 14, with the exception that the medicinal formulation was 0.02% w/w pirbuterol acetate in HFA-227 propellant. The amount of pirbuterol acetate adsorbed to the valve was 36 (+/-13) µg measured according to the method described above. Visual observation of the inside surface of the metering tanks showed considerably less drug deposition than the corresponding metering tanks from Comparative Example 9 using untreated valves.

### Comparative Example 9

Metered dose inhalers were prepared as in Example 16, with the exception that the metering valves used were untreated. The amount of pirbuterol acetate adsorbed to the valve was 43 (+/-12) µg measured according to the method described above.

### Example 17

MDIs were prepared as in Example 14, with the exception that the medicinal formulation was 0.02% w/w flunisolide in HFA-227 propellant. The amount of flunisolide adsorbed to the valve was 23 (+/-7) µg measured according to the method described above. Visual observation of the inside surface of the metering tanks showed considerably less drug deposition than the corresponding metering tanks from Comparative Example 10 using untreated valves.

### Comparative Example 10

Metered dose inhalers were prepared as in Example 17, with the exception that the metering valves used were untreated. The amount of flunisolide adsorbed to the valve was 41 (+/-7) µg measured according to the method described above.

The present invention has been described with reference to several embodiments thereof. The foregoing detailed description and examples have been provided for clarity of understanding only, and no unnecessary limitations are to be understood therefrom. It will be apparent to those skilled in the art that many changes can be made to the described embodiments without departing from the scope of the invention. Thus, the scope of the invention should not be limited to the exact details of the compositions and structures described herein, but rather by the language of the claims that follow.

## Claims

1. A container suitable for use with a propellant-based medicinal formulation, the container comprising one or more treated metal surfaces that come into contact with the medicinal formulation, wherein the one or more treated metal surfaces are **characterized by** an organic surface treatment compound covalently bonded to the metal surface through a reactive head group selected from either phosphonic acid or carboxylic acid and wherein the organic surface treatment compound is further **characterized by** an exposed, substantially non-reactive tail group disposed away from the metal surface.

2. A container as claimed in claim 1, wherein the tail group comprises 3 to 22 carbons.

3. A container as claimed in claim 1 or 2, wherein the tail group comprises fluorine.

4. A container as claimed in claim 3, wherein the tail group terminates with a linear or branched perfluoroalkyl having 1 to 4 carbons.

5. A container as claimed in claim 1, wherein the organic surface treatment compound has the formula X-R₁-R₂,
wherein X is 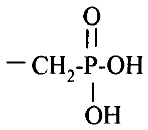 or -COOH;
R₁ is linear or branched alkyl having 0 to 22 carbons and optionally substituted by -O-;
R₂ is linear or branched perfluoroalkyl having 0 to 6 carbons; and
wherein R₁ and R₂ together comprise at least 3 and no more than 22 carbons.

6. A container as claimed in claim 5, wherein X is

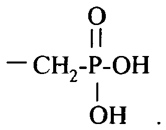

7. A container as claimed in claim 5 or 6, wherein R₁ has 0 carbons.

8. A container as claimed in claim 5 or 6, wherein R₂ has 0 carbons.

9. A container as claimed in any one of claims 5, 6, or 8, wherein R₁ is linear or branched alkyl having 3 to 22 carbons.

10. A container as claimed in any one of claims 6, 7, or 9, wherein R₂ is linear or branched perfluoroalkyl having 4 carbons.

11. A container as claimed in any preceding claim, wherein substantially all of the organic surface treatment compound is covalently bound to the one or more treated metal surfaces.

12. A container as claimed in any preceding claim, wherein adjacent organic surface treatment compounds are not crosslinked to each other.

13. A metered dose inhaler comprising a container as claimed in any one of claims 1 to 12.

14. A metered dose inhaler as claimed in claim 13, wherein the one or more treated metal surfaces comprise a valve.

15. A metered dose inhaler valve comprising a metering chamber, a stem, and a spring, one or more of which are metal and wherein at least one metal surface of the valve is **characterized by** an organic surface treatment compound covalently bonded to the metal surface through a reactive head group selected from either phosphonic acid or carboxylic acid and wherein the organic surface treatment compound is further **characterized by** an exposed, substantially non-reactive tail group disposed away from the metal surface.

16. A method of preparing a container suitable for use with a propellant-based medicinal formulation comprising the steps of:
a) providing the container having at least one metal surface;
b) providing a treatment solution comprising an organic surface treatment compound comprising a reactive head group selected from either phosphonic acid or carboxylic acid and a substantially non-reactive tail group;
c) applying the treatment solution to the metal surface of the container;
d) allowing the treatment solution to remain in contact with the metal surface of the container for a period of time sufficient to allow the reactive head group to covalently bond with the metal surface;
e) removing the treatment solution from contact with the metal surface and optionally rinsing any non-covalently bound organic surface treatment compound from the metal surface; and
f) drying the container, thereby providing a container having a metal surface **characterized by** an organic surface treatment compound covalently bonded to the metal surface through a reactive head group and wherein the organic surface treatment compound is further **characterized by** a substantially non-reactive tail group disposed away from the metal surface and wherein at least a portion of the non-reactive tail group is exposed to air.

## Patentansprüche

1. Behälter, geeignet für die Verwendung mit einer treibmittelbasierten medizinischen Formulierung, wobei der Behälter eine oder mehrere behandelte Metalloberflächen umfasst, die mit der medizinischen Formulierung in Kontakt kommen, wobei die eine oder mehreren behandelten Metalloberflächen **gekennzeichnet sind durch** eine organische Oberflächenbehandlungsverbindung, die über eine reaktionsfähige Kopfgruppe, ausgewählt aus Phosphonsäure und Carbonsäure, kovalent an die Metalloberfläche gebunden ist, und wobei die organische Oberflächenbehandlungsverbindung ferner **gekennzeichnet ist durch** eine exponierte, im Wesentlichen nicht reaktionsfähige Schwanzgruppe, die von der Metalloberfläche weg gerichtet ist.

2. Behälter gemäß Anspruch 1, wobei die Schwanzgruppe 3 bis 22 Kohlenstoffe umfasst.

3. Behälter gemäß Anspruch 1 oder 2, wobei die Schwanzgruppe Fluor umfasst.

4. Behälter gemäß Anspruch 3, wobei die Schwanzgruppe mit einem linearen oder verzweigten Perfluoralkyl mit 1 bis 4 Kohlenstoffen endet.

5. Behälter gemäß Anspruch 1, wobei die organische Oberflächenbehandlungsverbindung die Formel X-R₁-R₂ aufweist,
wobei X 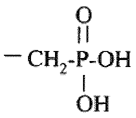 oder -COOH ist;
R₁ lineares oder verzweigtes Alkyl mit 0 bis 22 Kohlenstoffen, gegebenenfalls substituiert mit -O-, ist;
R₂ lineares oder verzweigtes Perfluoralkyl mit 0 bis 6 Kohlenstoffen ist; und
wobei R₁ und R₂ zusammen wenigstens 3 und nicht mehr als 22 Kohlenstoffe umfassen.

6. Behälter gemäß Anspruch 5, wobei X 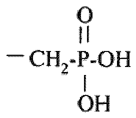 ist.

7. Behälter gemäß Anspruch 5 oder 6, wobei R₁ 0 Kohlenstoffe aufweist.

8. Behälter gemäß Anspruch 5 oder 6, wobei R₂ 0 Kohlenstoffe aufweist.

9. Behälter gemäß einem der Ansprüche 5, 6 oder 8, wobei R₁ lineares oder verzweigtes Alkyl mit 3 bis 22 Kohlenstoffen ist.

10. Behälter gemäß einem der Ansprüche 6, 7 oder 9, wobei R₂ lineares oder verzweigtes Perfluoralkyl mit 4 Kohlenstoffen ist.

11. Behälter gemäß einem der vorstehenden Ansprüche, wobei im Wesentlichen die gesamte organische Oberflächenbehandlungsverbindung kovalent an die eine oder mehreren behandelten Metalloberflächen gebunden ist.

12. Behälter gemäß einem der vorstehenden Ansprüche, wobei benachbarte organische Oberflächenbehandlungsverbindungen nicht miteinander vernetzt sind.

13. Dosierinhalator, umfassend einen Behälter gemäß einem der Ansprüche 1 bis 12.

14. Dosierinhalator gemäß Anspruch 13, wobei die eine oder mehreren behandelten Metalloberflächen ein Ventil umfassen.

15. Dosierinhalatorventil, umfassend eine Dosierkammer, einen Bolzen und eine Feder, wovon eines oder mehrere aus Metall sind und wobei wenigstens eine Metalloberfläche des Ventils **gekennzeichnet ist durch** eine organische Oberflächenbehandlungsverbindung, die über eine reaktionsfähige Kopfgruppe, ausgewählt aus Phosphonsäure und Carbonsäure, kovalent an die Metalloberfläche gebunden ist, und wobei die organische Oberflächenbehandlungsverbindung ferner **gekennzeichnet ist durch** eine exponierte, im Wesentlichen nicht reaktionsfähige Schwanzgruppe, die von der Metalloberfläche weg gerichtet ist.

16. Verfahren zum Herstellen eines Behälters, der für die Verwendung mit einer treibmittelbasierten medizinischen Formulierung geeignet ist, umfassend die Schritte:
a) Bereitstellen des Behälters mit wenigstens einer Metalloberfläche;
b) Bereitstellen einer Behandlungslösung, umfassend eine organische Oberflächenbehandlungsverbindung, die eine reaktionsfähige Kopfgruppe, ausgewählt aus Phosphonsäure und Carbonsäure, und eine im Wesentlichen nicht reaktionsfähige Schwanzgruppe umfasst;
c) Aufbringen der Behandlungslösung auf die Metalloberfläche des Behälters;
d) in Kontakt bleiben lassen der Behandlungslösung mit der Metalloberfläche des Behälters über einen ausreichenden Zeitraum, um das kovalente Binden der reaktionsfähigen Kopfgruppe an die Metalloberfläche zu erlauben;
e) Entfernen der Behandlungslösung aus dem Kontakt mit der Metalloberfläche und gegebenenfalls Abspülen von nicht kovalent gebundener organischer Oberflächenbehandlungsverbindung von der Metalloberfläche; und
f) Trocknen des Behälters, um so einen Behälter mit einer Metalloberfläche bereitzustellen, die **gekennzeichnet ist durch** eine organische Oberflächenbehandlungsverbindung, die über eine reaktionsfähige Kopfgruppe kovalent an die Metalloberfläche gebunden ist, und wobei die organische Oberflächenbehandlungsverbindung ferner **gekennzeichnet ist durch** eine im Wesentlichen nicht reaktionsfähige Schwanzgruppe, die von der Metalloberfläche weg gerichtet ist und wobei wenigstens ein Teil der nicht reaktionsfähigen Schwanzgruppe gegenüber Luft exponiert ist.

## Revendications

1. Récipient approprié pour être utilisé avec une formulation médicinale à base de propulseur, le récipient comprenant une ou plusieurs surfaces métalliques traitées qui viennent en contact avec la formulation médicinale, dans lequel la ou les surfaces métalliques traitées sont **caractérisées par** un composé de traitement de surface organique lié de façon covalente à la surface métallique par un groupe de tête réactif choisi entre soit un acide phosphonique soit un acide carboxylique et dans lequel le composé de traitement de surface organique est en outre **caractérisé par** un groupe de queue pratiquement non réactif exposé disposé à distance de la surface métallique.

2. Récipient selon la revendication 1, dans lequel le groupe de queue comprend 3 à 22 atomes de carbone.

3. Récipient selon la revendication 1 ou 2, dans lequel le groupe de queue comprend du fluor.

4. Récipient selon la revendication 3, dans lequel le groupe de queue se termine par un perfluoroalkyle linéaire ou ramifié ayant 1 à 4 atomes de carbone.

5. Récipient selon la revendication 1, dans lequel le composé de traitement de surface organique répond à la formule X-R₁-R₂,
dans laquelle
X est 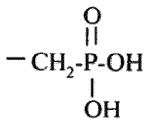 ou -COOH ;
R₁ est un alkyle linéaire ou ramifié ayant 0 à 22 atomes de carbone et facultativement substitué par -O- ;
R₂ est un perfluoroalkyle linéaire ou ramifié ayant 0 à 6 atomes de carbone ; et dans laquelle
R₁ et R₂ comprennent ensemble au moins 3 et pas plus de 22 atomes de carbone.

6. Récipient selon la revendication 5, dans lequel X est

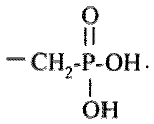

7. Récipient selon la revendication 5 ou 6, dans lequel R₁ a 0 atome de carbone.

8. Récipient selon la revendication 5 ou 6, dans lequel R₂ a 0 atome de carbone.

9. Récipient selon l'une quelconque des revendications 5, 6 ou 8, dans lequel R₁ est un alkyle linéaire ou ramifié ayant 3 à 22 atomes de carbone.

10. Récipient selon l'une quelconque des revendications 6, 7 ou 9, dans lequel R₂ est un perfluoroalkyle linéaire ou ramifié ayant 4 atomes de carbone.

11. Récipient selon l'une quelconque des revendications précédentes, dans lequel pratiquement tout le composé de traitement de surface organique est lié de façon covalente à la ou les surfaces métalliques traitées.

12. Récipient selon l'une quelconque des revendications précédentes, dans lequel des composés de traitement de surface organiques adjacents ne sont pas réticulés les uns avec les autres.

13. Aérosol-doseur comprenant un récipient selon l'une quelconque des revendications 1 à 12.

14. Aérosol-doseur selon la revendication 13, dans lequel la ou les surfaces métalliques traitées comprennent une valve.

15. Valve d'aérosol-doseur comprenant une chambre de dosage, une tige et un ressort, un ou plusieurs de ceux-ci étant métalliques et dans laquelle au moins une surface métallique de la valve est **caractérisée par** un composé de traitement de surface organique lié de façon covalente à la surface métallique par un groupe de tête réactif choisi entre soit un acide phosphonique soit un acide carboxylique et dans laquelle le composé de traitement de surface organique est en outre **caractérisé par** un groupe de queue pratiquement non réactif exposé disposé à distance de la surface métallique.

16. Procédé de préparation d'un récipient approprié pour être utilisé avec une formulation médicinale à base de propulseur comprenant les étapes consistant à :
a) obtenir le récipient ayant au moins une surface métallique ;
b) obtenir une solution de traitement comprenant un composé de traitement de surface organique comprenant un groupe de tête réactif choisi entre soit un acide phosphonique soit un acide carboxylique et un groupe de queue pratiquement non réactif ;
c) appliquer la solution de traitement sur la surface métallique du récipient ;
d) laisser la solution de traitement rester en contact avec la surface métallique du récipient pendant une durée suffisante pour permettre au groupe de tête réactif de se lier de façon covalente avec la surface métallique ;
e) enlever la solution de traitement du contact avec la surface métallique et facultativement rincer de la surface métallique tout composé de traitement de surface organique non lié de façon covalente ; et
f) sécher le récipient,
ce qui permet d'obtenir de cette manière un récipient ayant une surface métallique **caractérisée par** un composé de traitement de surface organique lié de façon covalente à la surface métallique par un groupe de tête réactif et dans lequel le composé de traitement de surface organique est en outre **caractérisé par** un groupe de queue pratiquement non réactif disposé à distance de la surface métallique et dans lequel au moins une partie du groupe de queue non réactif est exposée à l'air.
